# EUROPEAN PATENT APPLICATION

(11) **EP 4 699 608 A1**
(43) Date of publication of application: **25.02.2026**
(21) Application number: 24811164.3
(22) Date of filing: 23.05.2024
(51) Int. Cl.: A61K 31/4545, A61P 25/02, A61P 25/04, A61P 29/00

(54) **COMPOSITION FOR PREVENTING OR TREATING CANCER PAIN**

(30) Priority: 25.05.2023 JP 2023086153
(71) Applicant: Maruishi Pharmaceutical Co., Ltd., Osaka 538-0042 (JP)
(72) Inventor: KUBO Erika, Osaka-shi, Osaka 538-0042 (JP); YAMAMURA Yoshiro, Osaka-shi, Osaka 538-0042 (JP); SHIMADA Shoichi, Suita-shi, Osaka 565-0871 (JP); YAMAMOTO Yukiko, Suita-shi, Osaka 565-0871 (JP)
(74) Representative: Witte, Weller & Partner Patentanwälte mbB
(86) International application number: PCT/JP2024/018940
(87) International publication number: WO 2024/242158

(57) **Abstract**

An object of the present invention is to provide a preventive or therapeutic drug for cancer pain. The present invention provides a pharmaceutical composition for preventing or treating cancer pain, comprising a compound represented by the following formula (I): 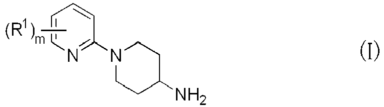 or a pharmaceutically acceptable salt thereof and a pharmaceutically acceptable carrier,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

## Description

### TECHNICAL FIELD

The present invention relates to a composition for preventing or treating cancer pain.

### BACKGROUND ART

Cancer is one of the three major diseases that rank high as a cause of death in Japan. The symptoms of cancer vary depending on the type of cancer and the affected site, but typically include cancer pain. Cancer pain is reported to occur in 70 to 80% of patients with advanced cancer.

To improve the QOL (Quality of Life) of cancer patients, relief of cancer pain is believed to be essential (Non-Patent Literature 1).

In drug therapy for cancer pain, selecting analgesics according to the order as described in the WHO three-step pain relief (analgesic) ladder is fundamental, and analgesics are selected based on the intensity of pain. As the therapy is escalated to the second and third steps, opioids are mainly used for drug treatment. Opioids are, however, known to cause various side effects, including dependence (psychological and physical dependence), tolerance (analgesic tolerance), and drowsiness.

Many drugs other than opioids at a single dose show no effect on cancer pain in cancer pain animal models, as previously reported (Non-Patent Literatures 2 to 7). Pregabalin, a non-opioid analgesic, has been reported to be effective in cancer pain animal models, but is known to have side effects, including sedative effects such as dizziness, drowsiness, and loss of consciousness (Non-Patent Literatures 2 and 8). Non-Patent Literature 9 describes that the coordinated motor ability and the spontaneous locomotor activity of rats were evaluated using the rotarod method and the Supermex method to assess the influences of pregabalin on the central nervous system, indicating that pregabalin suppresses the functions of the central nervous system (inhibits coordinated motor ability) at a dose lower than that is required to exert an analgesic effect (Table 2 in Non-Patent Literature 9).

Under such circumstances, the development of effective therapeutic drugs for cancer pain is desired.

1-(6-Chloropyridin-2-yl)piperidin-4-amine (also referred to as "compound A" below) is known to serve as a serotonin 3 receptor agonist (Patent Literatures 1 and 2). Patent Literature 3 discloses a pharmaceutical composition for preventing or treating chronic pain, comprising a serotonin 3 receptor agonist, including compound A, and a pharmaceutically acceptable carrier. Chronic pain is defined as "pain that lasts beyond the expected period of time required for healing, or pain associated with progressive non-cancerous diseases" (Non-Patent Literature 1). The above composition is not known to be effective for cancer pain. Further, the serotonin 3 receptor agonist as well as selective serotonin reuptake inhibitors (SSRIs) and serotonin noradrenaline reuptake inhibitors (SNRIs), which are in common with the serotonin 3 receptor agonist in that serotonin is involved, have been reported to have no effect on cancer pain in cancer pain animal models (Non-Patent Literatures 2 and 3).

### CITATION LIST

### NON-PATENT LITERATURE

Non-Patent Literature 1: Iryoyomayaku Tekiseishiyo Guidance issued by Compliance and Narcotics Division in Pharmaceutical Safety and Environmental Health Bureau of Ministry of Health, Labour and Welfare, April 2017.
Non-Patent Literature 2: Mohammad Zafar Imam et al, Assessment of the anti-allodynic efficacy of a glycine transporter 2 inhibitor relative to pregabalin and duloxetine in a rat model of prostate cancer-induced bone pain, Pharmacological Reports, July 26, 2020, vol. 72, pp. 1418-1425.
Non-Patent Literature 3: Mohammed El Mouedden et al, Pharmacological evaluation of opioid and non-opioid analgesics in a murine bone cancer model of pain, Pharmacology, Biochemistry and Behavior, January 12, 2007, vol. 86, pp. 458-467.
Non-Patent Literature 4: Janette Nallely Corona-Ramos et al, The effect of Gabapentin and Tramadol in Cancer Pain Induced by Glioma Cell in Rat Femur, Drug Development Research, 2017, vol. 78, pp. 173-183.
Non-Patent Literature 5: Simeng Ma et al, Amitriptyline influences the mechanical withdrawal threshold in bone cancer pain rats by regulating glutamate transporter GLAST, Molecular Pain, 2019, vol. 15, pp. 1-12.
Non-Patent Literature 6: Tansy Donovan-Rodriguez et al, Gabapentin Normalizes Spinal Neuronal Responses That Correlate with Behavior in a Rat Model of Cancer-induced Bone Pain, Anesthesiology, 2005, vol. 102, pp. 132-140.
Non-Patent Literature 7: Osamu Saito et al, Analgesic effects of nonsteroidal antiinflammatory drugs, acetaminophen, and morphine in a mouse model of bone cancer pain, Journal of Anesthesia, 2005, vol. 19, pp. 218-224.
Non-Patent Literature 8: Viatris Inc., Package insert of LYRICA (registered trademark) capsules and OD tablets 25 mg, 75 mg, and 150 mg, revised on July 2022 (third edition).
Non-Patent Literature 9: Yutaka Kitano, et al., Folia Pharmacologica Japonica, 2019, vol. 154, pp. 352-361.

Patent Literature 1: JP S56-36481 A
Patent Literature 2: JP H7-145166 A
Patent Literature 3: JP Pat. No. 7090344

### SUMMARY OF INVENTION

### TECHNICAL PROBLEM

An object of the present invention is to provide a preventive or therapeutic drug for cancer pain.

### SOLUTION TO PROBLEM

The inventors conducted extensive studies to solve the above problems and found that a compound represented by the general formula (I) below has an excellent effect of preventing or treating cancer pain, and completed the present invention.

That is, the present invention relates to a composition as described below.
[1] A pharmaceutical composition for preventing or treating cancer pain, comprising a compound represented by the following formula (I): 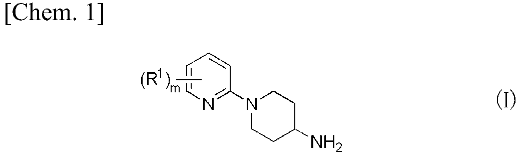 or a pharmaceutically acceptable salt thereof and a pharmaceutically acceptable carrier,
   wherein m represents an integer of 1 to 4; and
   each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.
[2] The pharmaceutical composition according to the above [1], wherein each R¹ is independently a halogen atom.
[3] The pharmaceutical composition according to the above [1] or [2], wherein each R¹ is a chlorine atom.
[4] The pharmaceutical composition according to any one of the above [1] to [3], wherein m is 1.
[5] The pharmaceutical composition according to any one of the above [1] to [4], wherein the compound represented by the formula (I) or a pharmaceutically acceptable salt thereof is a compound represented by the following formula (I'): 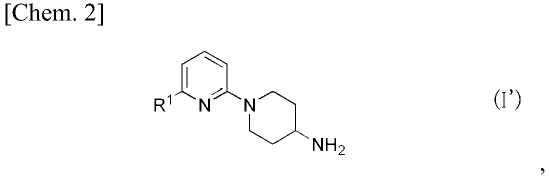
   wherein R¹ has the same meaning as defined above,
   or a pharmaceutically acceptable salt thereof.
[6] The pharmaceutical composition according to any one of the above [1] to [5], wherein the compound represented by the formula (I) or a pharmaceutically acceptable salt thereof is a compound represented by the following formula: 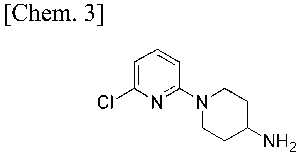 or a pharmaceutically acceptable salt thereof.
[7] An agent for preventing or treating cancer pain, comprising a compound represented by the above formula (I) or a pharmaceutically acceptable salt thereof,
   wherein m and R¹ in the formula (I) have the same meaning as defined in any one of the above [1] to [4].
[8] An agent for increasing a cancer pain threshold, comprising a compound represented by the above formula (I) or a pharmaceutically acceptable salt thereof,
   wherein m and R¹ in the formula (I) have the same meaning as defined in any one of the above [1] to [4].
[9] The agent according to the above [7] or [8], wherein the compound represented by the formula (I) or a pharmaceutically acceptable salt thereof is compound A or a pharmaceutically acceptable salt thereof.
[10] A method for preventing or treating cancer pain, comprising administering an effective amount of a compound represented by the above formula (I) or a pharmaceutically acceptable salt thereof to a human or a non-human animal,
   wherein m and R¹ in the formula (I) have the same meaning as defined in any one of the above [1] to [4].
[11] A method for increasing a cancer pain threshold, comprising administering an effective amount of a compound represented by the above formula (I) or a pharmaceutically acceptable salt thereof to a human or a non-human animal,
   wherein m and R¹ in the formula (I) have the same meaning as defined in any one of the above [1] to [4].
[12] The method according to the above [10] or [11], wherein the compound represented by the formula (I) or a pharmaceutically acceptable salt thereof is compound A or a pharmaceutically acceptable salt thereof.
[13] Use of a compound represented by the above formula (I) or a pharmaceutically acceptable salt thereof in production of a pharmaceutical composition for preventing or treating cancer pain or an agent for preventing or treating cancer pain,
   wherein m and R¹ in the formula (I) have the same meaning as defined in any one of the above [1] to [4].
[14] Use of a compound represented by the above formula (I) or a pharmaceutically acceptable salt thereof in production of a pharmaceutical composition for increasing a cancer pain threshold or an agent for increasing a cancer pain threshold,
   wherein m and R¹ in the formula (I) have the same meaning as defined in any one of the above [1] to [4].
[15] The use according to the above [13] or [14], wherein the compound represented by the formula (I) or a pharmaceutically acceptable salt thereof is compound A or a pharmaceutically acceptable salt thereof.
[16] Use of a compound represented by the above formula (I) or a pharmaceutically acceptable salt thereof for preventing or treating cancer pain,
   wherein m and R¹ in the formula (I) have the same meaning as defined in any one of the above [1] to [4].
[17] Use of a compound represented by the above formula (I) or a pharmaceutically acceptable salt thereof for increasing a cancer pain threshold,
   wherein m and R¹ in the formula (I) have the same meaning as defined in any one of the above [1] to [4].
[18] The use according to the above [16] or [17], wherein the compound represented by the formula (I) or a pharmaceutically acceptable salt thereof is compound A or a pharmaceutically acceptable salt thereof.
[19] A compound represented by the above formula (I) or a pharmaceutically acceptable salt thereof for use in preventing or treating cancer pain,
   wherein m and R¹ in the formula (I) have the same meaning as defined in any one of the above [1] to [4].
[20] A compound represented by the above formula (I) or a pharmaceutically acceptable salt thereof for use in increasing a cancer pain threshold,
   wherein m and R¹ in the formula (I) have the same meaning as defined in any one of the above [1] to [4].
[21] The compound or a pharmaceutically acceptable salt thereof for use according to the above [19] or [20], wherein the compound represented by the formula (I) or a pharmaceutically acceptable salt thereof is compound A or a pharmaceutically acceptable salt thereof.
[22] The pharmaceutical composition according to any one of the above [1] to [6], the agent according to any one of the above [7] to [9], the method according to any one of the above [10] to [12], the use according to any one of the above [13] to [18], or the compound or a pharmaceutically acceptable salt thereof for use according to any one of the above [19] to [21], wherein the cancer pain is at least one selected from pain directly caused by cancer itself, pain arising from cancer treatment, pain associated with cancer, and pain caused by a non-cancer related disease that is concurrently affecting a cancer patient.
[23] The pharmaceutical composition according to any one of the above [1] to [6], the agent according to any one of the above [7] to [9], the method according to any one of the above [10] to [12], the use according to any one of the above [13] to [18], or the compound or a pharmaceutically acceptable salt thereof for use according to any one of the above [19] to [21], wherein the cancer pain is at least pain directly caused by cancer itself.
[24] The pharmaceutical composition according to any one of the above [1] to [6], the agent according to any one of the above [7] to [9], the method according to any one of the above [10] to [12], the use according to any one of the above [13] to [18], or the compound or a pharmaceutically acceptable salt thereof for use according to any one of the above [19] to [21], wherein the cancer pain is at least pain directly caused by cancer itself, and further comprises one or more selected from pain arising from cancer treatment, pain associated with cancer, and pain caused by a non-cancer related disease that is concurrently affecting a cancer patient.

### ADVANTAGEOUS EFFECTS OF INVENTION

The present invention provides a preventive or therapeutic drug for cancer pain. The present invention also provides a preventive or therapeutic drug for cancer pain, wherein the drug preferably does not have side effects observed in the administration of pregabalin (e.g., sedative effect etc.) and/or side effects observed in the administration of an opioid (e.g., dependence (psychological and physical dependence), tolerance (analgesic tolerance), drowsiness, etc.). The present invention also exhibits a comparable preventive or therapeutic effect on cancer pain relative to morphine, preferably at a single dose. The present invention also exhibits a comparable preventive or therapeutic effect on cancer pain relative to morphine, preferably via oral administration.

### BRIEF DESCRIPTION OF DRAWINGS

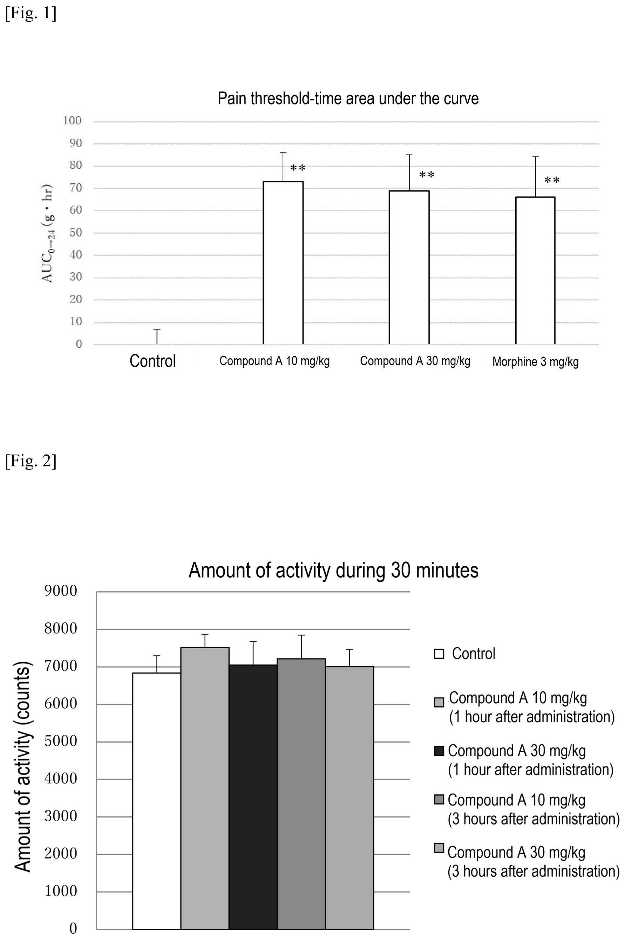

Fig. 1 is a graph showing the measurement result of the pain thresholds in cancer pain model rats. The values are means ± standard error (S.E.) (the number of animals in each group: 8). ** indicates that a significant difference was observed between the control group and the compound A treatment groups as determined by Dunnett's test with a critical value of 1%.
Fig. 2 is a graph showing the evaluation result of the influences (side effects) of compound A on the central nervous system based on changes in the spontaneous locomotor activity of mice after administration of compound A. The values are means ± standard error (S.E.) (the number of animals in each group: 8). No statistical difference was observed between the control group and the compound A treatment groups as determined using Dunnett's test with a critical value of 5%.

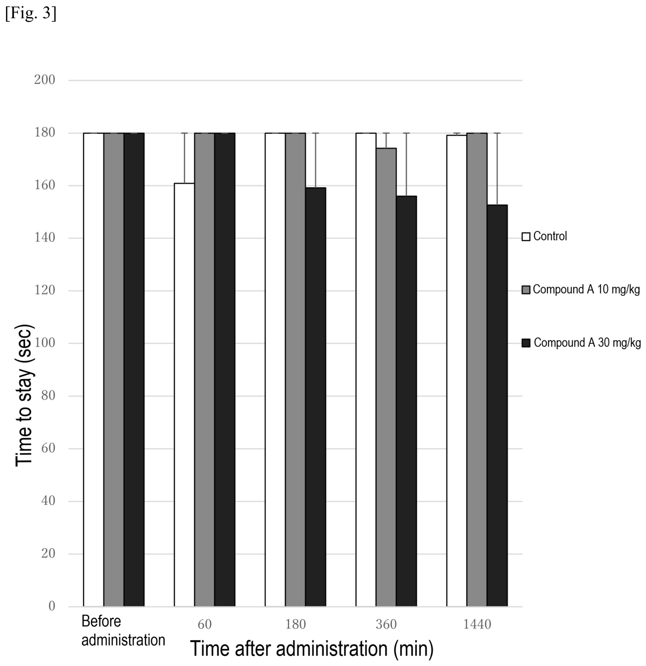

Fig. 3 is a graph showing the evaluation result of the influences (side effects) of compound A on the central nervous system based on changes in the coordinated motor ability of rats after administration of compound A. The values are means ± standard error (S.E.) (the number of animals in each group: 6). No statistically significant difference was observed between the control group and the compound A treatment groups as determined using Dunnett's test or Steel's test with a critical value of 5%.

### DESCRIPTION OF EMBODIMENTS

The present invention will be described in detail below.

The compound represented by the formula (I) below or a pharmaceutically acceptable salt thereof will be described in detail below: 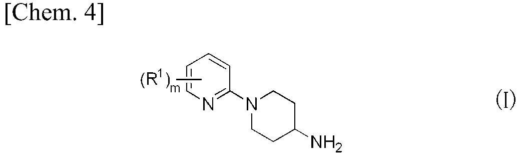
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

The "halogen atom" independently represents any of a fluorine atom, a chlorine atom, a bromine atom, and an iodine atom.

The term "optionally substituted" means that a moiety may not be substituted or may be substituted to have one or more substituents. In an exemplary case where a moiety is substituted, R¹ in the above general formula (I) is a methoxy group that is substituted with at least one or more (preferably 1 to 3) halogen atoms (e.g., fluorine atoms, chlorine atoms, bromine atoms, or iodine atoms) at a substitutable position(s).

Each symbol in the compound represented by the formula (I) will be described below.

In the formula (I), m is an integer of 1 to 4, and is preferably an integer of 1 to 3, more preferably an integer of 1 or 2, and even more preferably 1.

Each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

The halogen atom at R¹ is preferably a fluorine atom or a chlorine atom, and is particularly preferably a chlorine atom.

The methyl group optionally substituted with 1 to 3 halogen atoms at R¹ is preferably a methyl group optionally substituted with 1 to 3 fluorine atoms, and is particularly preferably a methyl group or a trifluoromethyl group.

The methoxy group optionally substituted with 1 to 3 halogen atoms at R¹ is preferably a methoxy group optionally substituted with 1 to 3 fluorine atoms, and is particularly preferably a methoxy group or a trifluoromethoxy group.

The methylthio group optionally substituted with 1 to 3 halogen atoms at R¹ is preferably a methylthio group optionally substituted with 1 to 3 fluorine atoms, and is particularly preferably a methylthio group or a trifluoromethylthio group.

In an embodiment, each R¹ is independently a halogen atom, and each R¹ is preferably a chlorine atom.

In another embodiment, m is 1. In another embodiment, R¹ is a halogen atom and m is 1.

In another embodiment, the compound represented by the formula (I) or a pharmaceutically acceptable salt thereof is a compound represented by the following formula (I'): 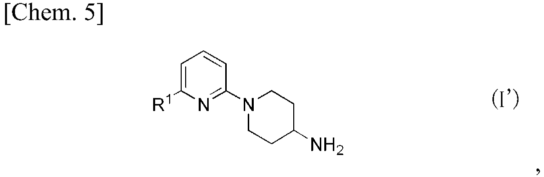 wherein R¹ has the same meaning as defined above,
or a pharmaceutically acceptable salt thereof.

In a preferred embodiment, the compound represented by the formula (I) is a compound represented by the following formula: 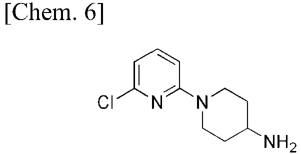 (i.e., compound A (compound name: 1-(6-chloropyridin-2-yl)piperidin-4-amine)).

The compound represented by the formula (I) or a pharmaceutically acceptable salt thereof can act as a serotonin 3 receptor agonist.

Examples of the pharmaceutically acceptable salt include acid addition salts, metal salts, base addition salts, and amino acid addition salts. Specific examples thereof include acid addition salts such as hydrochloric acid salts, hydrobromic acid salts, hydroiodic acid salts, sulfuric acid salts, nitric acid salts, phosphoric acid salts, formic acid salts, acetic acid salts, propionic acid salts, fumaric acid salts, oxalic acid salts, malonic acid salts, succinic acid salts, methanesulfonic acid salts, ethanesulfonic acid salts, benzenesulfonic acid salts, toluenesulfonic acid salts, maleic acid salts, dimaleic acid salts, lactic acid salts, malic acid salts, tartaric acid salts, citric acid salts, or trifluoroacetic acid salts; metal salts such as lithium salts, potassium salts, calcium salts, magnesium salts, sodium salts, zinc salts, or aluminum salts; base addition salts such as ammonium salts, diethanolamine salts, ethylenediamine salts, triethanolamine salts, or triethylamine salts; and amino acid addition salts such as those including glycine, lysine, arginine, ornithine, glutamic acid, or aspartic acid.

The compound represented by the formula (I) or a pharmaceutically acceptable salt thereof in the present invention includes inner salts and addition products thereof, solvates or hydrates thereof, and cocrystals thereof.

One or more compounds represented by the formula (I) or a pharmaceutically acceptable salt thereof in the present invention may be directly administered to a patient. If needed, a compound represented by the formula (I) or a pharmaceutically acceptable salt thereof can be combined with a pharmaceutically acceptable carrier, and provided as a formulation in a form well known to those skilled in the art.

Examples of such a carrier include, but are not limited to, commonly used carriers for pharmaceutical formulations, such as excipients, lubricants, binders, disintegrants, preservatives, pH adjusting agents, diluents, absorption enhancers, etc. Specific examples of excipients include sugar derivatives such as mannitol or sorbitol; starch derivatives such as corn starch or potato starch; cellulose derivatives such as crystalline cellulose; etc. Examples of lubricants include metallic stearates such as magnesium stearate; talc; etc. Examples of binders include hydroxypropyl cellulose, hydroxypropyl methylcellulose, polyvinylpyrrolidone, etc. Examples of disintegrants include cellulose derivatives such as carboxymethyl cellulose or carboxymethylcellulose calcium, etc. Examples of preservatives include p-hydroxybenzoate esters such as methylparaben or propylparaben; alcohols such as chlorobutanol or benzyl alcohol; etc. Examples of pH adjusting agents include inorganic acids such as hydrochloric acid, sulfuric acid, or phosphoric acid; organic acids such as acetic acid, succinic acid, fumaric acid, or malic acid; salts thereof; etc. Examples of diluents include water for injection, etc. These pharmaceutically acceptable carriers may be used alone or in combination of two or more.

The term "cancer pain" as used herein has the meaning commonly used in the art. The cancer pain is not particularly limited and may include all pain that a cancer patient may experience, including at least one selected from pain directly caused by cancer itself (e.g., tumor infiltration, growth, metastasis, etc.), pain arising from cancer treatment (e.g., postoperative pain, postoperative chronic pain, pain associated with nerve damage due to chemotherapy, etc.), pain associated with cancer (e.g., low back pain due to long-term bed rest, lymphedema, pressure ulcers, etc.), and pain caused by a non-cancer related disease that is concurrently affecting a cancer patient (e.g., osteoarthritis of the spine, migraine, etc.). Cancer pain may be continuous pain or breakthrough pain. Cancer pain may be pain responsive to opioids or pain not responsive to opioids. In a preferred embodiment of the present invention, cancer pain is pain including at least pain directly caused by cancer itself. In a more preferred embodiment of the present invention, cancer pain includes at least pain directly caused by cancer itself, and is pain including at least one selected from pain arising from cancer treatment (e.g., postoperative pain, postoperative chronic pain, pain associated with nerve damage due to chemotherapy, etc.), pain associated with cancer (e.g., low back pain due to long-term bed rest, lymphedema, pressure ulcers, etc.), and pain caused by a non-cancer related disease that is concurrently affecting a cancer patient (e.g., osteoarthritis of the spine, migraine, etc.).

The term "prevention" of pain as used herein includes the prevention of the onset of pain and the delay of the onset of pain in a subject at risk of developing pain.

The term "treatment" of pain as used herein means alleviating, relieving, improving, or partially or completely eliminating pain, suppressing the progression of the severity of pain, or the like in a subject suffering from pain. The term "effective amount" refers to a dose of a compound or a pharmaceutically acceptable salt thereof sufficient to achieve the purpose of the prevention or treatment. The effective amount can be explained with reference to, for example, the dosage and the number of doses as described below.

In an embodiment, the pharmaceutical composition of the present invention comprises a compound represented by the formula (I) or a pharmaceutically acceptable salt thereof. For the definitions of m and R¹ in the formula (I), reference may be made to the above definitions. In a preferred embodiment, the pharmaceutical composition of the present invention comprises a compound represented by the formula (I') or a pharmaceutically acceptable salt thereof. For the definitions of m and R¹ in the formula (I'), reference may be made to the above definitions. In a more preferred embodiment, the pharmaceutical composition of the present invention comprises compound A.

When the compound represented by the formula (I) of the present invention has an asymmetric carbon atom in the molecule, the compound represented by the formula (I) may exist as stereoisomers (i.e., diastereomers or optical isomers) at the asymmetric carbon atom. The active ingredient of the present invention includes any one of such stereoisomers or a mixture thereof.

The compound represented by the formula (I) or a pharmaceutically acceptable salt thereof in the present invention includes compounds labeled with an isotope (e.g., ²H, ³H, ¹³C, ¹⁴C, ¹⁵N, ¹⁸F, ³²P, ³⁵S, ¹²⁵I, etc.) and deuterated derivatives.

The compound represented by the formula (I) or a pharmaceutically acceptable salt thereof in the present invention can be administered orally or parenterally after combined with the above carrier as necessary. Specific examples of such oral formulations or parenteral formulations include tablets (e.g., sugar-coated tablets, film-coated tablets, sublingual tablets, orally disintegrating tablets, buccal tablets, etc.), pills, powders, granules, capsules (e.g., soft capsules, microcapsules, etc.), troches, syrups, solutions, emulsions, suspensions, controlled release formulations (e.g., fast release formulations, sustained release formulations, sustained release microcapsules, etc.), aerosols, films (e.g., orally disintegrating films, oral mucosal adhesive films, etc.), injections (e.g., subcutaneous injection, intravenous injection, intramuscular injection, intraperitoneal injection, etc.), intravenous infusions, transdermal formulations, ointments, lotions, patches, suppositories (e.g., rectal suppository, vaginal suppository, etc.), pellets, transnasal formulations, transpulmonary formulations (e.g., inhalants, etc.), eye drops, etc. Intrathecal administration is preferably excluded from the route of administration to reduce the burden on a subject animal. The compound represented by the formula (I) or a pharmaceutically acceptable salt thereof in the present invention is excellent in that the compound or a pharmaceutically acceptable salt thereof exhibits a preventive or therapeutic effect on cancer pain by a single dose of oral administration.

The compound represented by the formula (I) or a pharmaceutically acceptable salt thereof in the present invention that is formulated into any of the dosage form described above can be administered to, for example, a human or a non-human animal (e.g., a rat, a mouse, a rabbit, sheep, a pig, cattle, a cat, a dog, a monkey, etc.), preferably a human or a non-human mammal, more preferably a human, and especially preferably a human patient.

The dosage and the number of doses of the compound represented by the formula (I) or a pharmaceutically acceptable salt thereof in the present invention may vary as appropriate for conditions, including the severity of cancer pain, the age, body weight and sex of the patient, the type of drug, the dosage form, the route of administration, etc.

For parenteral administration to a mouse or a rat, the compound represented by the formula (I) as an active ingredient can be administered, for example, at about 0.1 to 10 mg/kg body weight per dose a day, preferably about 0.3 to 10 mg/kg body weight per dose a day, more preferably about 1 to 5 mg/kg body weight per dose a day, and especially preferably about 1 to 3 mg/kg body weight per dose a day by a parenteral route, such as a subcutaneous, intravenous, intraperitoneal, intramuscular, or rectal route. For oral administration, the compound represented by the formula (I) can be administered at about 1 to 100 mg/kg body weight per dose, preferably about 3 to 100 mg/kg body weight per dose, more preferably about 10 to 50 mg/kg body weight per dose, and especially preferably about 10 to 30 mg/kg body weight per dose.

For parenteral administration to a human, the compound represented by the formula (I) as an active ingredient can be administered, for example, at about 0.001 to 1 mg/kg body weight per dose a day, preferably about 0.005 to 0.3 mg/kg body weight per dose a day, and especially preferably about 0.01 to 0.1 mg/kg body weight per dose a day to a human adult by a parenteral route, such as a subcutaneous, intravenous, intraperitoneal, intramuscular, or rectal route. For oral administration, the compound represented by the formula (I) can be administered at about 0.01 to 10 mg/kg body weight per dose, preferably about 0.05 to 3 mg/kg body weight per dose, and especially preferably about 0.1 to 1 mg/kg body weight per dose.

The dose may be divided into one to several doses a day, for example, one to three doses a day, one or two doses a day, or a single dose a day.

A single compound represented by the formula (I) or a pharmaceutically acceptable salt thereof in the present invention is preferably administered alone, for example, compound A is administered alone. If needed, two or more compounds represented by the formula (I) may be administered in combination, and may further be administered in combination with another drug, such as another therapeutic agent for pain. Examples of such another therapeutic agent for pain include opioids, non-steroidal anti-inflammatory drugs, pregabalin, fluvoxamine, and a pharmaceutically acceptable salt thereof. Examples of the opioids include strong opioids and weak opioids. Specific examples of the opioids include codeine, morphine, oxycodone, fentanyl, remifentanil, meperidine, buprenorphine, pentazocine, pethidine, butorphanol, tramadol, naloxone, etc. Examples of the non-steroidal anti-inflammatory drugs include aspirin, ethenzamide, diflunisal, Loxonin, ibuprofen, diclofenac, ketoprofen, naproxen, piroxicam, indomethacin, etc.

When two or more compounds represented by the formula (I) or a pharmaceutically acceptable salt thereof in the present invention are used in combination and/or when the compound(s) represented by the formula (I) or a pharmaceutically acceptable salt thereof is used in combination with another drug, the compound(s) and/or the drug may be administered simultaneously, sequentially, or separately. The compound(s) and/or the drug may be formulated as a combination drug containing two or more drugs or may be formulated as separate compositions.

The compound represented by the formula (I) or a pharmaceutically acceptable salt thereof in the present invention may be a commercially available product, or may be produced by a known method or a method known per se. For example, the compound represented by the formula (I) or a pharmaceutically acceptable salt thereof can be produced by the method described in Patent Literature 1 or 2. Compound A may be a commercially available product.

Whether the compound represented by the formula (I) or a pharmaceutically acceptable salt thereof in the present invention can be used for the prevention or treatment of cancer pain can be evaluated as follows. For example, a cancer pain model animal can be used to compare a group with administration of the compound represented by the formula (I) or a pharmaceutically acceptable salt thereof in the present invention with a group with no administration of the compound or a pharmaceutically acceptable salt thereof by measuring a pain threshold (e.g., 1, 3, 6, and 24 hours after administration) in a conventionally known testing system selected as appropriate from known methods. Preferably, a tendency to significance or a significant difference is observed between the two groups based on known statistical methods, and more preferably a significant difference is observed between the two groups. The compound represented by the formula (I) or a pharmaceutically acceptable salt thereof in the present invention can also be used as an agent for increasing a cancer pain threshold (e.g., 1, 3, or 6 hours after administration).

The compound represented by the formula (I) or a pharmaceutically acceptable salt thereof can be formulated by appropriately blending the active ingredient, i.e., the compound represented by the formula (I) or a pharmaceutically acceptable salt thereof, with a pharmaceutically acceptable carrier and an additive as necessary according to a known production method for pharmaceutical formulations (e.g., the method as described in the Japanese Pharmacopoeia). The blending ratio of the active ingredient and the additive may be appropriately set based on the range commonly used in the pharmaceutical field. The additive that can be blended with the active ingredient is not particularly limited, and examples thereof include various additives, such as a colorant, a flavor improver, and a fragrance.

The term "about" as used herein is intended to include, for example, a slight deviation. The term may refer to, for example, a value within ± 10%, ± 5%, ± 2%, ± 1%, ± 0.5%, ± 0.2%, ± 0.1%, or ± 0.05%. Such ranges may also include experimental errors inherent in standard methods used for the measurement and/or quantification of a given value or range.

The present invention includes aspects in which the configurations described above are combined in various ways to achieve the effects of the present invention without departing from the technical scope of the present invention.

### EXAMPLES

The present invention will be described more specifically with reference to Examples. The present invention is not limited to these Examples, and various modifications and production techniques based on the fundamental technology of the present invention can be implemented by a person who has ordinary knowledge in the art and will be explained below.

### Experiment 1: Pain treatment effects of compound A and pre-existing drug

### Experimental method

Five-week-old female Crl:CD(SD) SPF rats (Charles River Japan) were injected with MRMT-1 cells (rat breast cancer cells) at 3 × 10⁴ cells/20 µL/ body into the medullary cavity at the proximal end of the diaphysis of the right tibia to create a bone metastasis model (day 0). On day 10 after the transplantation of the cancer cells (day 10), the rats were divided into 4 groups of 8 rats each: a control group (with administration of distilled water (trade name: Otsuka Distilled Water) manufactured by Otsuka Pharmaceutical Factory, Inc.), a 10 mg/kg compound A administration group, a 30 mg/kg compound A administration group, and a morphine administration group, as shown in Table 1 below. On day 11 after the transplantation of the cancer cells (day 11), each drug was orally or subcutaneously administered to the rats at a dose of 2 mL/kg. The pain threshold (g) of the plantar surface of the right hind paw was measured using a Dynamic Plantar Aesthesiometer (37450, Ugo Basile) under blinded conditions 1, 3, 6, and 24 hours after the administration. The average value of the pain threshold-time area under the curve (AUC) (g·hr) for 24 hours post-administration was determined for each group.

**Table 1**

| Test Group | Dose (mg/kg) | Administration Route | Number of Animals |
|---|---|---|---|
| Control | 0 | Oral | 8 |
| Compound A 10 mg/kg | 10 | | 8 |
| Compound A 30 mg/kg | 30 | | 8 |
| Morphine 3 mg/kg | 3 | Subcutaneous | 8 |

### Result of Experiment 1

The result is shown in Fig. 1. The cancer pain threshold significantly increased in the 10 mg/kg and 30 mg/kg compound A administration groups compared to that in the control group, indicating that compound A exhibited an analgesic effect. The pain threshold-time area under the curve (AUC) (g·hr) of these two groups showed no statistically significant difference compared to that of the group with subcutaneous administration of morphine up to 24 hours after administration.

### Experiment 2: Evaluation of influences (side effects) of compound A on central nervous system based on changes in spontaneous locomotor activity and coordinated motor ability after administration of compound A

### Experimental method

### (1) Measurement of spontaneous locomotor activity

Five- to six-week-old male ICR-SPF mice (Japan SLC, Inc.) were divided into 5 groups of 8 mice each: a control group (with administration of distilled water (trade name: Otsuka Distilled Water) manufactured by Otsuka Pharmaceutical Factory, Inc.), two 10 mg/kg compound A administration groups, and two 30 mg/kg compound A administration groups, as shown in Table 2 below. Compound A or distilled water at a dose of 10 mL/kg was orally administered to the mice. One or three hours after the administration of compound A or distilled water, the mice were transferred to a measurement cage, and spontaneous locomotor activity was measured for 30 minutes using Supermex (registered trademark) (Muromachi Kikai Co., Ltd.). The result is shown in Fig. 2.

**Table 2**

| Test Group | Number of animals |
|---|---|
| Control | 8 |
| Compound A 10 mg/kg (1 hour after administration) | 8 |
| Compound A 30 mg/kg (1 hour after administration) | 8 |
| Compound A 10 mg/kg (3 hours after administration) | 8 |
| Compound A 30 mg/kg (3 hours after administration) | 8 |

### (2) Measurement of coordinated motor ability

Five- to six-week-old male SD-SPF rats (Japan SLC, Inc.) were used in this experiment. The experimental schedule included training using a rotarod (SN445, Shinano Seisakusyo) for 3 days (6 times a day), starting from 3 days before the day of administration. On the day of administration, pre-values (time to stay on the rotarod before the administration of compound A or distilled water) were measured twice, and the rats that spent 180 seconds or more on the rotarod in either of the two measurements were selected. The rats were divided into 3 groups of 6 rats each: a control group (with administration of distilled water (trade name: Otsuka Distilled Water) manufactured by Otsuka Pharmaceutical Factory, Inc.), a 10 mg/kg compound A administration group, and a 30 mg/kg compound A administration group, as shown in Table 3 below. Compound A or distilled water was orally administered to the rats at a dose of 10 mL/kg, and the time to stay on the rotarod was measured 1, 3, 6, and 24 hours after the administration under the test conditions of 10 rotations/min with a cutoff of 180 seconds. The result is shown in Fig. 3.

**Table 3**

| Test Group | Number of animals |
|---|---|
| Control | 6 |
| Compound A 10 mg/kg | 6 |
| Compound A 30 mg/kg | 6 |

### Results of Experiment 2

As is apparent from the result shown in Fig. 2, no significant changes in the amount of activity were observed in either of the compound A administration groups compared to that in the control group. This result indicates that either of the compound A administration groups showed no significant changes in the spontaneous locomotor activity compared to that in the control group.

As is apparent from the result shown in Fig. 3, no significant changes in the coordinated motor ability were observed in either of the compound A administration groups compared to that in the control group.

These results from Experiment 2 demonstrate that the administration of compound A to mice and rats at 10 to 30 mg/kg does not suppress the spontaneous locomotor activity or the coordinated motor ability.

### Summary of results

The results from Experiments 1 and 2 reveal that compound A does not suppress spontaneous locomotor activity or coordinated motor ability at a dose that induces an analgesic effect. This result suggests that compound A is useful as an active ingredient for an analgesic agent for ingestion without causing influences (side effects, such as sedation) on the central nervous system.

### INDUSTRIAL APPLICABILITY

The composition of the present invention is useful as a pharmaceutical composition for preventing or treating cancer pain.

## Claims

1. A pharmaceutical composition for preventing or treating cancer pain, comprising a compound represented by the following formula (I): 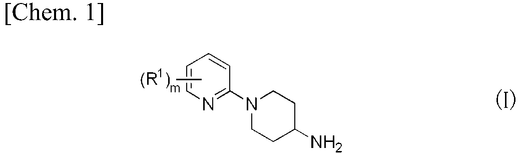 or a pharmaceutically acceptable salt thereof and a pharmaceutically acceptable carrier,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

2. The pharmaceutical composition according to claim 1, wherein each R¹ is independently a halogen atom.

3. The pharmaceutical composition according to claim 1 or 2, wherein each R¹ is a chlorine atom.

4. The pharmaceutical composition according to any one of claims 1 to 3, wherein m is 1.

5. The pharmaceutical composition according to any one of claims 1 to 4, wherein the compound represented by the formula (I) or a pharmaceutically acceptable salt thereof is a compound represented by the following formula (I'): 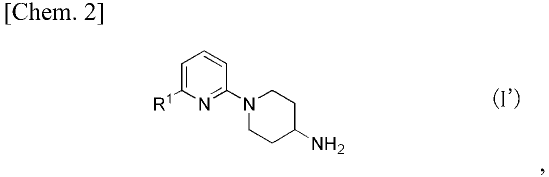 wherein R¹ has the same meaning as defined above,
or a pharmaceutically acceptable salt thereof.

6. The pharmaceutical composition according to any one of claims 1 to 5, wherein the compound represented by the formula (I) or a pharmaceutically acceptable salt thereof is a compound represented by the following formula: 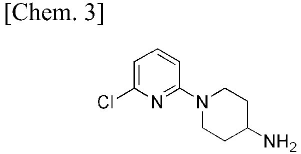 or a pharmaceutically acceptable salt thereof.

7. An agent for preventing or treating cancer pain, comprising a compound represented by the following formula (I): 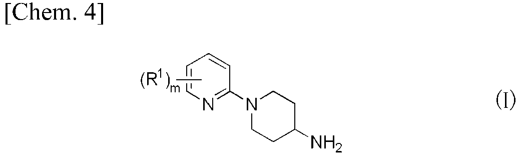 or a pharmaceutically acceptable salt thereof,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

8. An agent for increasing a cancer pain threshold, comprising a compound represented by the following formula (I): 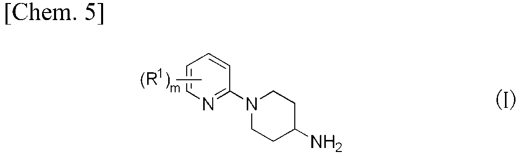 or a pharmaceutically acceptable salt thereof,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

9. A method for preventing or treating cancer pain, comprising administering an effective amount of a compound represented by the following formula (I): 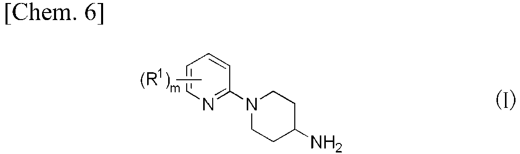 or a pharmaceutically acceptable salt thereof to a human or a non-human animal,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

10. A method for increasing a cancer pain threshold, comprising administering an effective amount of a compound represented by the following formula (I): 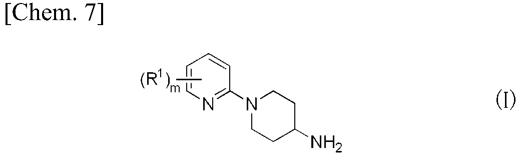 or a pharmaceutically acceptable salt thereof to a human or a non-human animal,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

11. Use of a compound represented by the following formula (I): 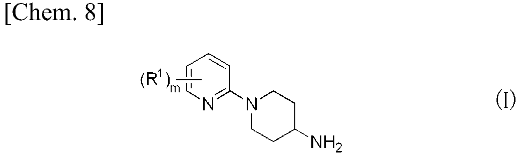 or a pharmaceutically acceptable salt thereof in production of a pharmaceutical composition for preventing or treating cancer pain or an agent for preventing or treating cancer pain,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

12. Use of a compound represented by the following formula (I): 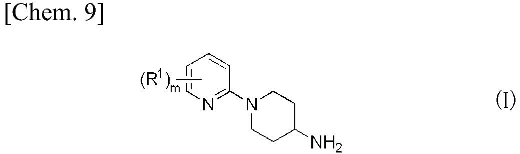 or a pharmaceutically acceptable salt thereof in production of a pharmaceutical composition for increasing a cancer pain threshold or an agent for increasing a cancer pain threshold,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

13. Use of a compound represented by the following formula (I): 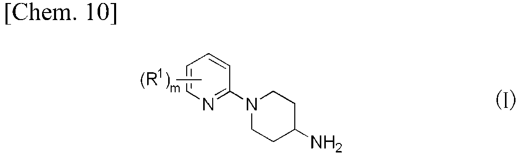 or a pharmaceutically acceptable salt thereof for preventing or treating cancer pain,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

14. Use of a compound represented by the following formula (I): 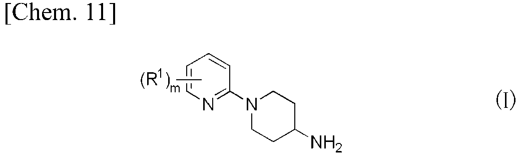 or a pharmaceutically acceptable salt thereof for increasing a cancer pain threshold,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

15. A compound represented by the following formula (I): 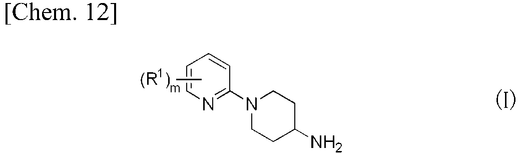 or a pharmaceutically acceptable salt thereof for use in preventing or treating cancer pain,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

16. A compound represented by the following formula (I): 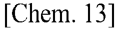 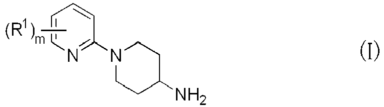 or a pharmaceutically acceptable salt thereof for use in increasing a cancer pain threshold,
wherein m represents an integer of 1 to 4; and
each R¹ is independently selected from the group consisting of a hydrogen atom, a halogen atom, a methyl group optionally substituted with 1 to 3 halogen atoms, a methoxy group optionally substituted with 1 to 3 halogen atoms, and a methylthio group optionally substituted with 1 to 3 halogen atoms.

17. The pharmaceutical composition according to any one of claims 1 to 6, the agent according to claim 7 or 8, the method according to claim 9 or 10, the use according to any one of claims 11 to 14, or the compound or a pharmaceutically acceptable salt thereof for use according to claim 15 or 16, wherein the cancer pain is at least one selected from pain directly caused by cancer itself, pain arising from cancer treatment, pain associated with cancer, and pain caused by a non-cancer related disease that is concurrently affecting a cancer patient.
